# EUROPÄISCHE PATENTSCHRIFT

(11) **EP 1 030 616 B1**
(45) Veröffentlichungstag und Bekanntmachung des Hinweises auf die Patenterteilung: **23.07.2003**
(21) Anmeldenummer: 98966147.5
(22) Anmeldetag: 05.11.1998
(51) Int. Cl.: A61B 17/80

(54) **IMPLANTAT ZUR STABILISIERUNG EINER FRAKTUR UND SCHRAUBE ZUR VERWENDUNG IN DER CHIRURGIE**
IMPLANT FOR STABILIZING A FRACTURE AND SCREW FOR USE IN SURGERY
IMPLANT POUR LA STABILISATION D'UNE FRACTURE ET VIS A UTILISER EN CHIRURGIE

(30) Priorität: 14.11.1997 DE 19750493
(43) Veröffentlichungstag der Anmeldung: 30.08.2000
(73) Patentinhaber: Mückter, Helmut, 52076 Aachen (DE)
(72) Erfinder: Mückter, Helmut, 52076 Aachen (DE)
(74) Vertreter: Castell, Klaus, Dr.
(86) Internationale Anmeldenummer: DE9803303
(87) Internationale Veröffentlichungsnummer: WO99025266

(56) Entgegenhaltungen:
- DE-U- 9 200 328
- US-A- 2 612 159
- US-A- 4 465 065
- US-A- 4 657 001
- US-A- 5 693 055
- "Osteosynthesis Traumatology Orthopaedics" November 1996 , OSTEO AG , SELZACH, SCHWEIZ XP002096040 22291 "T-plates" siehe Seite 1.14

## Beschreibung

Die Erfindung betrifft ein Implantat zur Stabilisierung einer Fraktur des Oberarmes im Bereich des Oberarmkopfes mit oder ohne Beteiligung der Tubercula mit einer Platte, in der Bohrungen für Schrauben angeordnet sind, wobei die Platte eine Führung für eine Oberarmkopfschraube aufweist, die die Oberarmkopfschraube stabil in einem Winkel zur Platte hält.

Als gattungsgemäßes Implantat ist aus der US 5,693,055 eine Unterleg- und Führungsscheibe bekannt, welche die Kräfte am Schraubenkopf aufnimmt und durch eine vergrößerte Auflagefläche auf dem Schaftknochen des Oberarmes eine geringere Spannung im Knochenschaft erreichen soll. Diese Führungsplatte liegt auf dem Schaftknochen im Sinne einer Unterlegscheibe auf und wird lediglich durch die im Oberarmkopf eingebrachte Schraube gegen den Schaftknochen fixiert. Die Funktion des im US-Patent 5,693,055 beschriebenen Implantates ist somit auf die Schraubenkraft im Oberarmkopf und die hiermit erzeugte interfragmentäre Kompression angewiesen, welche jedoch erfahrungsgemäß in Folge einer regelmäßig einsetzenden Knochensinterung innerhalb von Stunden bis Tagen nachläßt.

Ein anderes Implantat ist aus der US 4,657,001 bekannt. Dieses Implantat ist jedoch nur für den Oberschenkelbereich geeignet und führt ebenfalls zu einer interfragmentären Kompression.

Der Erfindung liegt daher die Aufgabe zugrunde, ein gattungsgemäßes Implantat so weiterzubilden, daß mit ihm eine einfache, sichere Stabilisierung der Fraktur zu erzielen ist.

Diese Aufgabe wird dadurch gelöst, daß die Platte eine Einspannvorrichtung aufweist, die die Oberarmkopfschraube stabil gegen eine Verschiebung in Schaftrichtung vorzugsweise lösbar fixiert.

Eine derartige Einspannvorrichtung blockiert Bewegungen der Schrauben relativ zur Platte und sorgt somit für ein starres Halteimplantat, das sich auch bei starken Bewegungen des Oberarmes nicht lockern kann.

Bei der Verwendung von zwei Oberarmkopfschrauben wird nicht nur eine winkelstabile Fixierung von Kopf- und Schaftfragment des Knochens zueinander gewährleistet, sonder darüber hinaus wird zusätzlich eine Rotationsstabilität erzielt, welche ein Auslockern der Oberarmkopfschrauben, z. B. bei Pendelübungen verhindert.

Ein Ausfuhrungsbeispiel sieht vor, daß die Führungen Bohrungsteile sind, deren Querschnitte einem Schaftdurchmesser der Oberarmkopfschrauben entsprechen. Diese Führungen nutzen die Plattendicke, um die Schrauben in einem Winkel zu halten. Hierzu wird der Innendurchmesser der Bohrung zumindest auf einem Bohrungsabschnitt genau an den Außendurchmesser eines Schaftbereiches der Schraube angepaßt, so daß die Schraube zwar in die Bohrung hineinsteckbar ist, danach aber nur noch in Richtung der Achse des Schraubenschaftes verschiebbar ist. Diese Ausgestaltung erfordert zwar eine gewisse Plattendicke, sie ist jedoch Führungen vorzuzichen, die über die Platten oberfläche vorstehen oder umgekehrt in den Knochen hineinragen.

Der von den Führungen vorgebene Winkel sollte etwa dem physiologischen Winkel des Oberarmkopfes entsprechen. Daher wird ein Winkel zwischen 35° und 40°, vorzugsweise 37,5°, gemessen zur Längsachse des Oberarmschaftes, vorgeschlagen.

Eine bevorzugte Ausgestaltung einer Einspannvorrichtung sieht vor, daß zwischen den Bohrungen der Oberarmkopfschrauben ein Spalt mit einer Querbohrung angebracht ist. In diese Querbohrung kann eine Schraube derart eingebracht werden, daß durch Anziehen der Schraube der Spalt verengt wird. Dies führt dazu, daß der Durchmesser der Bohrungen leicht verformt wird und dadurch die Oberarmkopfschrauben unter Beibehaltung ihrer Winkellage fixiert werden.

Vorzugsweise sind die Bohrungen der Oberarmkopfschrauben parallel zueinander so angeordnet, daß ihre Achsen mit der Längsachse des Oberarmschaftes in einer Ebene liegen. Die Achsen der Oberarmkopfschrauben kreuzen somit die Längsachse des Oberarmschaftes in dem vorgegebenen Winkel in vorzugsweise 35°- 40°. Diese Anordnung der Schrauben auf einer Linie ermöglicht eine Operation mit einer sehr schmalen Freilegung des Knochens im Frakturbereich. Dies fuhrt zu einer schnellen knöchernen Heilung, da Durchblutungsstörungen vermieden werden.

Um Drahtcerclagen oder PDS-Kordeln zur Refixation ausgerissener Tubercula aufzunehmen, wird vorgeschlagen, an der Platte parallel zu ihrer Ober- und Unterseite verlaufende Bohrungen anzubringen. Diese Bohrungen sind vorzugsweise an der Plattenspitze und im Bereich der Einspannvorrichtung vorgesehen.

Eine bevorzugte Ausgestaltung der Platte ist sehr kompakt gestaltet. Diese Platte hat eine maximale Länge von 100 mm, eine maximale Breite von 12 mm und ihre Dicke liegt zwischen 4 mm und 9.5 mm. Eine besondere Gestaltung der Schraubenführung erlaubt eine Plattendicke von maximal etwa 7 mm. In ihrer Formgebung ist die Platte den anatomischen Verhältnissen im Bereich des proximalen Humerus angepaßt. Im unteren Abschnitt wird die Platte mit handelsüblichen Corticalisschrauben am proximalen Anteil des Oberarmschaftes verschraubt. Da in diesem gelenknahen Bereich keine hohen Biegemomente auftreten, ist eine Fixierung mit drei Schrauben ausreichend. Zur Versorgung von Frakturen, welche in den Oberarmschaftbereich einstrahlen, kann die Platte auch länger mit entsprechend erhöhter Anzahl an Schraubenbohrungen im Bereich des Oberarmschaftes ausgeführt werden.

Verschiedene Ausführungsbeispiele der Erfindung sind in der Zeichnung dargestellt und werden im folgenden näher beschrieben.

Es zeigt
- Figur 1: einen Längsschnitt durch die mit Schrauben im Oberarmknochen befestigte Platte,
- Figur 2: mehrere Ansichten der herausgezeichneten Platte mit einer Spannbackenschraube,
- Figur 3: als Einzelheit die Positionierung der Spannbackenschraube,
- Figur 4: eine alternative Möglichkeit zum Verspannen der Oberarmkopfschraube im Bereich des Plattensitzes,
- Figur 5: eine weitere Alternative zur Verspannung mittels eines Schraubenkopfgewindes,
- Figur 6: eine Oberarmkopfschraube,
- Figur 7: einen Ausschnitt aus dem Gewinde der Oberarmkopfschraube nach Figur 6,
- Figur 8: einen Längsschnitt durch eine alternative Ausführungsform einer mit Schrauben im Oberarmknochen befestigten Platte und
- Figur 9: eine vergrößerte Einzelheit aus Figur 8.

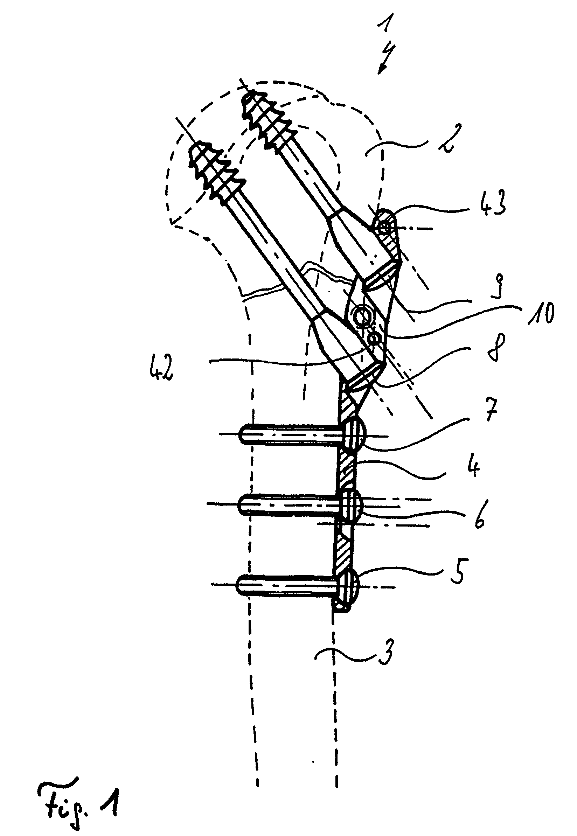

Die Figur 1 zeigt einen Oberarmknochen mit proximaler Humerusfraktur, Typ III nach Neer. Der abgebildete Teil des gebrochenen Oberarms besteht aus dem Oberarmkopf 2 und dem proximalen Anteil des Oberarmschaftes 3. Die Platte 4 liegt am proximalen Humerus an und ist in ihrer Formgebung an die anatomischen Verhältnisse im Bereich des proximalen Humerus angepaßt. Die Gesamtlänge der Platte beträgt 95 mm, ihre Dicke liegt im Bereich des proximalen Anteils des Oberarmschaftes bei 4 mm und ist im Bereich des Oberarmkopfes auf 9,5 mm verstärkt. Die Breite der Platte beträgt maximal 12 mm.

Diese Platte ist im unteren Abschnitt mit handelsüblichen Corticalisschrauben 5, 6, 7 am proximalen Anteil 3 des Oberarmschaftes verschraubt. Aufnahmen für spezielle Oberarmkopfschrauben 8 und 9 sind im oberen Abschnitt der Platte 4 als Spannbacken 10 ausgeführt, die eine feste Verklemmung der Oberarmkopfschrauben 8, 9 in der Platte 4 ermöglichen.

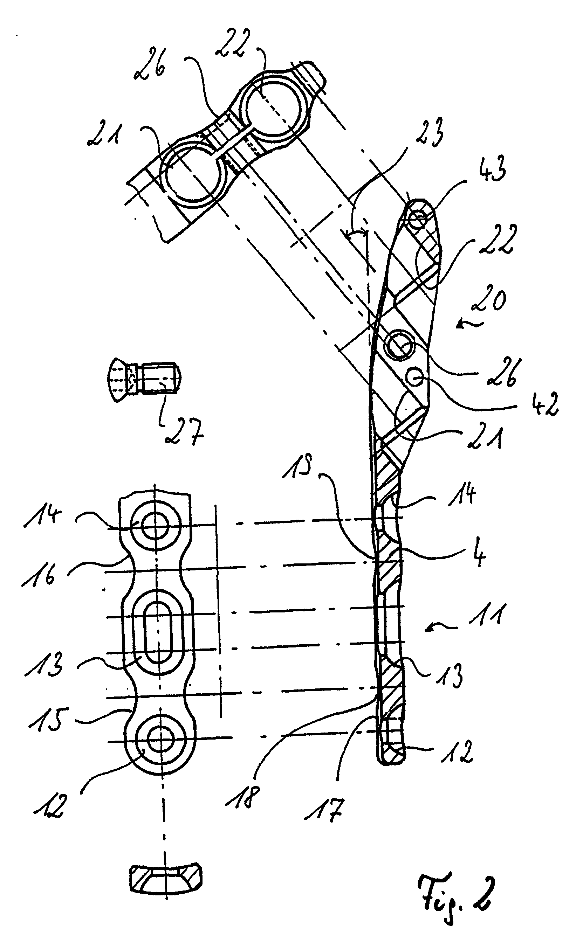

Die Figur 2 zeigt die herausgezeichnete Platte 4 in maßstabsgetreuer Darstellung. Im unteren Abschnitt 11 der Platte 4 sind auf einer Linie drei Bohrungen 12, 13, 14 angebracht, von denen die mittlere Bohrung 13 als Langloch ausgeführt ist. Diese Bohrungen sind zur Aufnahme von handelsüblichen Corticalisschrauben 5, 6, 7 ausgebildet. Zwischen diesen Bohrungen weist die Platte Einschnürungen 15 und 16 auf, wodurch eine gleichmäßigere Spannungsverteilung in der Platte erzielt wird. Die Anlagefläche 17 ist nicht eben, sondern konkav ausgeführt, wodurch die Platte besser an der Oberfläche des Oberarmknochen anliegt.

Die Linien 18 und 19 stellen die Sichtkanten der Einschnürungen 15 und 16 dar, welche aufgrund der konkaven Formgebung der Platte zur Darstellung kommen. Es handelt sich hierbei also nicht um Vertiefungen, die Platte liegt flächig auf.

Der obere Teil 20 der Platte 4 weist Führungen 21, 22 als Aufnahme für die Oberarmkopfschrauben 8 und 9 auf. Diese Führungen sind in einem Winkel 23 von 37,5° gegenüber der Oberarmlängsachse angebracht. Dies entspricht etwa dem physiologischen Winkel des Oberarmkopfes. Diese Führungen sind als Bohrungen ausgebildet, die einen größeren Durchmesser zur Aufnahme eines Schraubenkopfes 24 und einen etwas kleineren Durchmesser zur Aufnahme eines verdickten Schraubenschaftes 25 aufweisen. Die Tiefe der Führungen 21, 22 und damit die Dicke der Platte 4 in diesem Bereich ist so gewählt, daß die Länge der Führung gerade ausreicht, der Schraube 8 bzw. 9 einen sicheren Halt in einer vorbestimmten Winkellage zu geben.

Die in Figur 2 gezeigte Draufsicht auf den oberen Teil 20 der Platte 4 zeigt, daß zwischen den als Führung dienenden Bohrungen 21, 22 die Platte 4 einen Spalt aufweist. Quer zu diesem Spalt ist eine Bohrung 26 vorgesehen, in die eine Spannbackenschraube 27 eingeschraubt werden kann.

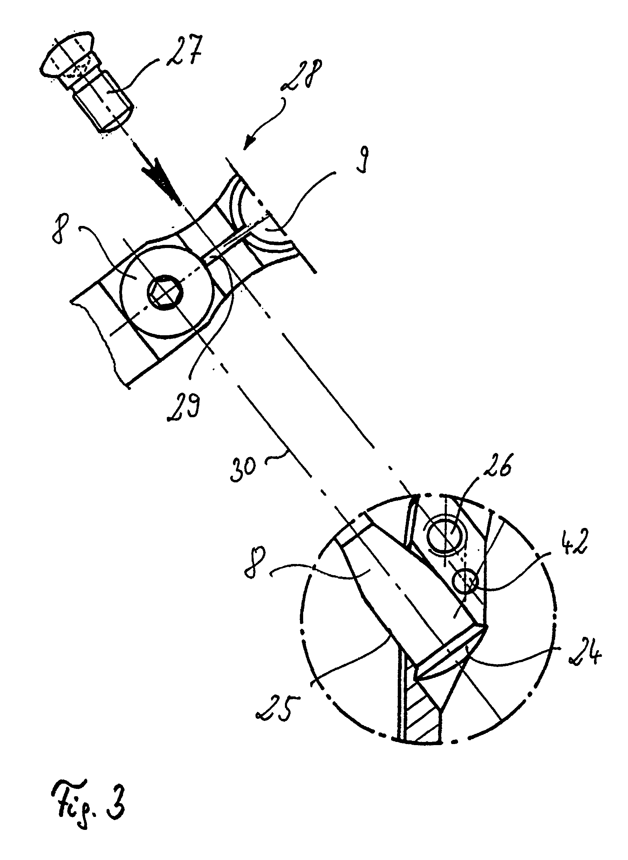

Die Einspannung der Schrauben 8 und 9, und insbesondere deren verdickten Teiles 25 mittels einer Spannbackeneinrichtung 28 zeigt Figur 3. Durch Eindrehen der Spannbackenschrauben 27 in das Gewinde der Bohrung 26 werden die beidseitig des Spaltes 29 liegenden Teile der Platte 4 aufeinander zubewegt, so daß sich der Spalt 29 verengt. Dadurch werden auch die Querschnitte der Führungen 21, 22 geringfügig verformt, so daß die Wandungen der Führungen an den verdickten Schaftteil 25 der Schrauben 8 bzw. 9 gedrückt werden. Dadurch werden die Schrauben 8 und 9 zusätzlich zur fixierten Winkellage gegen Verdrehung um die Schraubenachse 30 und Verschiebung in Richtung der Schraubenachse 30 fixiert.

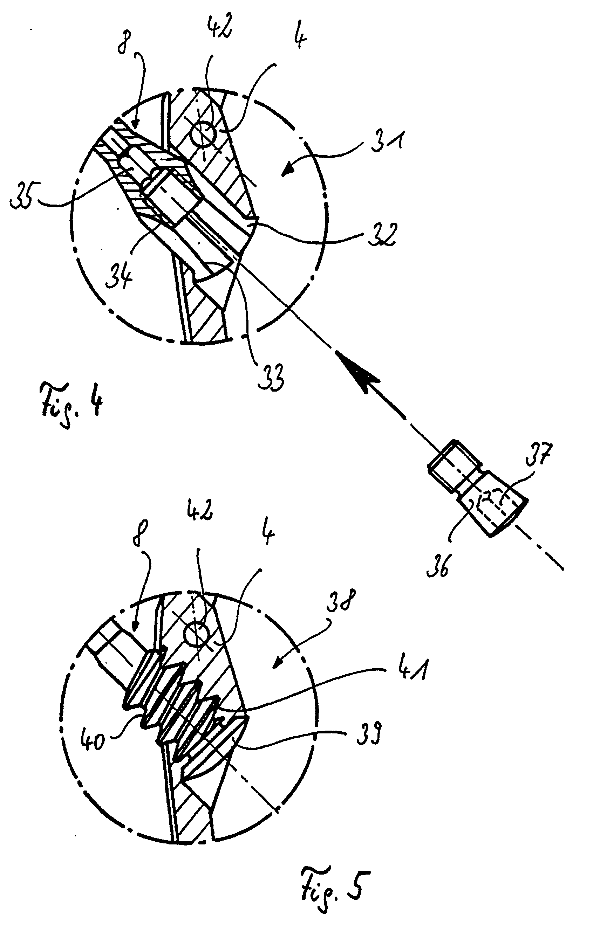

Eine alternative Einspannvorrichtung 31 zeigt die Figur 4. Bei dieser Art der Verspannung der Oberarmkopfschrauben 8 bzw. 9 in der Platte 4 ist der Schraubenkopf 32 geschlitzt und innen mit einer konischen Bohrung 33 und einem Gewinde 34 versehen. Am inneren Ende des Gewindes 34 schließt sich in der Schraube ein Innensechskant 35 an, mit dem die Schraube 8 in den Oberarmkopf eingeschraubt werden kann. Nach dem Eindrehen der Schraube 8 wird die Konusschraube 36 ebenfalls mit einem Innensechskant 37 in das Gewinde 33 der Schraube 8 eingeschraubt. Dadurch wird der geschlitzte Kopf 32 der Oberarmkopfschraube 8 auseinander gedrückt und damit in der Platte 4 fest verklemmt.

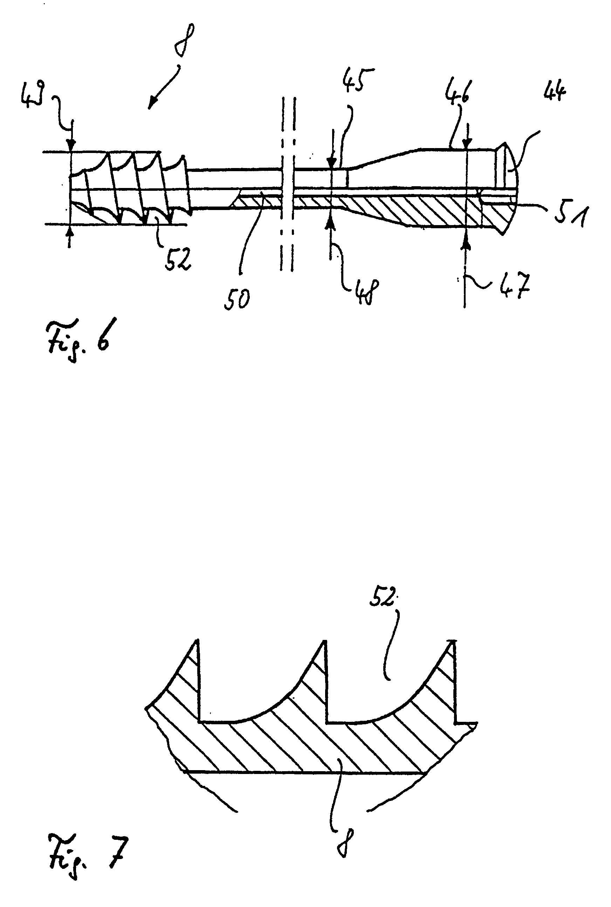

Eine weitere Alternative einer Einspannvorrichtung 38 zeigt die Figur 5. Bei dieser Art der Verspannung der Oberarmkopfschraube 8 ist der an den Schraubenkopf 39 angrenzende Schaft als Außengewinde 40 und die als Führung dienende Schraubenaufnahme 41 in der Platte 4 als entsprechendes Innengewinde ausgeführt. Der lichte Durchmesser des Gewindes in der Platte 4 ist mindestens so groß wie der Durchmesser des Spongiosagewindes der Schraube 8 am Schraubenende. Die Steigungen des Schraubenkopfgewindes 40 und des Spongiosagewindes am Schraubenende müssen etwa gleich sein, damit die Oberarmkopfschraube 8 eingeführt werden kann. Außerdem muß die Gewindesteigung 40 so ausgelegt sein, daß Selbsthemmung gewährleistet ist. Beim Eindrehen der Oberarmkopfschraube 8 wird diese schließlich fest angedreht, wodurch eine sichere Verspannung der Oberarmkopfschraube 8 in der Platte 4 erzielt wird.

Diese Variante gestattet im Vergleich zu den beiden zuvor beschriebenen Varianten keine bzw. nur eine minimale interfragmentäre Kompression. Dadurch wirkt sie funktionell wie ein Fixateur interne des Oberarmkopfes.

Quer zum Verlauf der beschriebenen Führungen der Oberarmkopfschrauben sind in der Platte Bohrungen 42, 43 vorgesehen, die der Aufnahme von Drahtcerclagen oder PDS-Kordeln zur Refixation ausgerissener Tubercula dienen.

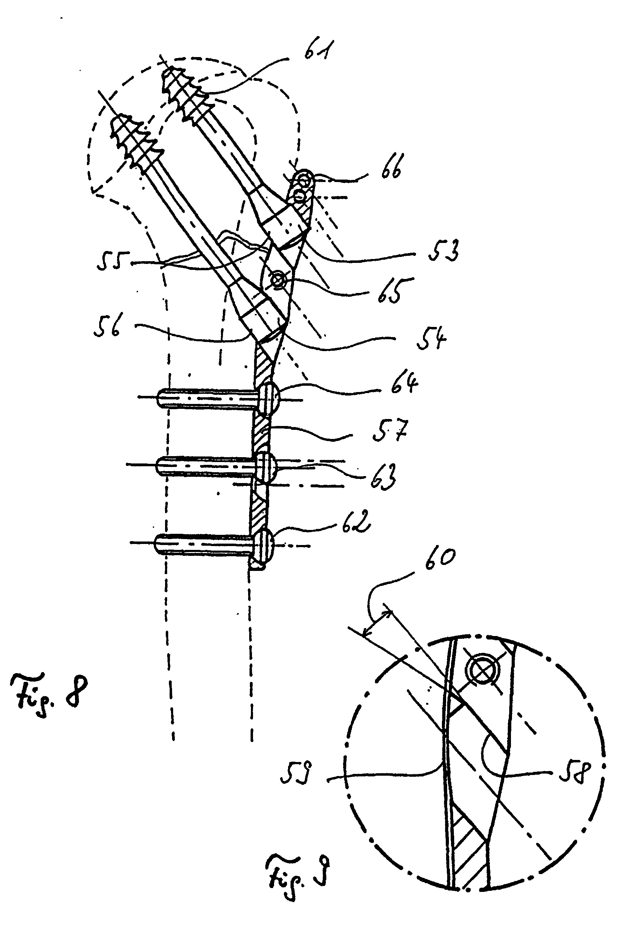

Eine Schraube 8, die in ihrem Aufbau bis auf die Länge der Schraube 9 entspricht, ist in Figur 6 dargestellt. Diese Schraube besteht aus dem Schraubenkopf 44, dem Schraubenschaft 45 und dem Gewinde 52. Der Schraubenschaft 45 ist zum Kopf 44 hin zunächst konisch ausgebildet, so daß er sich im Bereich des Kopfes 44 verdickt. Dieser, über eine Schaftstrecke ausgebildete verdickte Schaftteil 46 hat einen Schaftdurchmesser 47, der gegenüber dem übrigen Schaftdurchmesser 48 vergrößert ist. Der Außendurchmesser 49 des Gewindes 52 ist größer als der Durchmesser 48 des Schaftteils 45. Er ist jedoch gleichgroß oder kleiner als der Durchmesser 47 des verdickten Schaftteils 46. Die Schraube 8 weist eine zentrale Bohrung 50 auf, damit sie über ein spezielles Führungsdrahtsystem plaziert werden kann und im Schraubenkopf 44 ist ein Innensechskant 51 zum Festdrehen der Schraube 8 vorgesehen.

Um eine Anpassung der erforderlichen Schraubenlänge zu gewährleisten, sind die Schraubenlängen dieser Schrauben zwischen 40 mm und 80 mm in 5 mm Intervallen vorgesehen. Dies erlaubt es, für unterschiedliche Brüche und unterschiedliche Knochen, die jeweils richtige Schraubenlänge auszuwählen.

Die Schrauben sind mit einem Spongiosagewinde versehen. Eine Darstellung eines derartigen Gewindes ist in Figur 7 abgebildet. Für den erfindungsgemäßen Einsatzbereich wird nicht die handelsübliche Schraube mit 6,5 mm - Spongiosagewinde, sondern eine Schrauben mit 8,5 mm - Spongiosagewinde vorgesehen. Diese Schraube führt zu deutlich erhöhten Haltekräften im Knochen und ist daher auch für die Verwendung bei stark osteoporotisch verändertem Knochen geeignet.

Die in Figur 8 gezeigte Plattenkonstruktion entspricht im wesentlichen der Plattenkonstruktion nach Figur 1. Im Bereich der Aufnahme der Oberarmkopfschrauben ist die Platte jedoch deutlich schmaler ausgeführt. Die Oberarmkopfschrauben 53 und 54 besitzen nicht mehr, wie in Figur 1 gezeigt, einen Linsenkopf, sondern enden mit dem Führungsschaft 55 bzw. 56. Um der Oberarmkopfschraube 53 bzw. 54 in der Platte 57 ein Widerlager zu bieten, ist, wie in Figur 9 gezeigt, die Bohrung 58 in der Platte 57 nicht durchgehend, sondern reduziert sich unmittelbar an der Anlage am Knochen 59 mit einem Winkel 60 von ca. 15°.

Durch die schräge Lage der Bohrung 58 in der Platte 57 entsteht somit eine "Nase", welche der Schraube 54 bzw. der Schraube 53 als Widerlager dient. Das Spongiosagewinde 61 der Oberarmkopfschrauben 53 bzw. 54 kann entweder einen so kleinen Außendurchmesser erhalten, daß es problemlos auch durch die verengte Bohrung 58 eingesteckt werden kann, oder "Nase" und Spongiosagewinde sind so aufeinander abgestimmt, daß die Oberarmkopfschraube problemlos im Bereich der Verengung der Bohrung 58 eingedreht werden kann.

Durch diese bevorzugte Ausführungsform ist die Platte 57 um ca. 25% bis 30% schmaler zu gestalten als die Platte 4. Ihre maximale Breite beträgt nur noch 7 mm. Im Vergleich dazu tragen die Corticalisschrauben 62, 63, 64 im Schaftteil der Platte 57 ca. 6 mm auf.

Konstruktionsbedingt wurde das Gewinde der Spannbackenschraube 65 von M 5 auf M 4 reduziert und die Bohrung 66 zur Aufnahme einer PDS-Kordel bzw. eines Drahtes an das obere Ende der Platte 57 verlagert.

## Patentansprüche

1. Implantat zur Stabilisierung einer Fraktur des Oberarms im Bereich des Oberarmkopfes mit oder ohne Beteiligung der Tubercula
- mit einer Platte (4),
- in der Bohrungen (12, 13, 14) für Schrauben (5,6,7) angeordnet sind, ***wobei*** die Platte eine Führung (21, 22) für eine Oberarmkopfschraube aufweist, die die Oberarmkopfschraube stabil in einem Winkel zur Platte hält, ***dadurch gekennzeichnet, daß*** die Platte (4) eine Einspannvorrichtung (27, 28, 31, 38) aufweist, die die Oberarmkopfschraube (8, 9) stabil gegen eine Verschiebung in Schaftrichtung vorzugsweise lösbar fixiert.

2. Implantat nach Anspruch 1, ***dadurch gekennzeichnet, daß*** die Platte (4) Führungen (21, 22) für vorzugweise zwei Oberarmkopfschrauben (8, 9) aufweist.

3. Implantat nach Anspruch 1 oder 2, ***dadurch gekennzeichnet, daß*** die Führungen Bohrungsteile sind, deren Querschnitte einem Schaftdurchmesser (47) der Oberkopfschrauben (8, 9) entsprechen.

4. Implantat nach einem der vorhergehenden Ansprüche, ***dadurch gekennzeichnet, daß*** der Winkel (2, 3) zwischen 35° und 40° liegt und vorzugsweise 37,5° beträgt.

5. Implantat nach einem der vorhergehenden Ansprüche, **dadurch *gekennzeichnet, daß*** die Platte als Einspannvorrichtung zwischen den Bohrungen (21, 22) der Oberarmkopfschrauben (8, 9) einen Spalt (29) mit einer Querbohrung (26) aufweist.

6. Implantat nach einem der vorhergehenden Ansprüche, ***dadurch gekennzeichnet, daß*** die Bohrungsachsen (21, 22) der Oberarmkopfschrauben (8, 9) in einer Ebene mit der Längsachse des Oberarmschaftes liegen.

7. Implantat nach einem der vorhergehenden Ansprüchem, ***dadurch gekennzeichnet, daß*** die Platte (4) parallel zu ihrer Ober- und Unterseite verlaufende Bohrungen (42, 43) aufweist.

8. Implantat nach einem der vorhergehenden Ansprüche, ***dadurch gekennzeichnet, daß*** die Platte (4) eine maximale Dicke von ca. 6 bis 8 mm aufweist.

9. Implantat nach einem der vorhergehenden Ansprüche, ***dadurch gekennzeichnet, daß*** die Platte (4) eine maximale Breite von 12 mm aufweist.

## Claims

1. An implant for stabilizing a fracture of the humerus in the area of the head of the humerus, with or without involvement of the tubercula,
- having a plate (4),
- in which there are boreholes (12, 13, 14) for screws (5, 6, 7), whereby the plate has a guide (21, 22) for a head-of-the-humerus screw, which holds the head-of-the-humerus screw in a stable position at an angle to the plate, **characterized in that** the plate (4) has a chucking device (27, 28, 31, 38) which secures the head-of-the-humerus screw (8, 9), preferably detachably, in a stable position to prevent displacement in the direction of the shaft.

2. The implant according to Claim 1, **characterized in that** the plate (4) has guides (21, 22) for preferably two head-of-the-humerus screws (8, 9).

3. The implant according to Claim 1 or 2, **characterized in that** the guides are borehole parts whose cross sections correspond to a shaft diameter (47) of the head-of-the-humerus screws (8, 9).

4. The implant according to one of the preceding claims, **characterized in that** the angles (2, 3) are between 35° and 40°, preferably 37.5°.

5. The implant according to one of the preceding claims, **characterized in that** the plate as the chucking device has a gap (29) with a transverse borehole (26) between the boreholes (21, 22) of the head-of-the-humerus screws (8, 9).

6. The implant according to one of the preceding claims, **characterized in that** the axes of the boreholes (21, 22) of the head-of-the-humerus screws (8, 9) are situated in a plane with the longitudinal axis of the shaft of the humerus.

7. The implant according to one of the preceding claims, **characterized in that** the plate (4) has boreholes (42, 43) running in parallel to its top and bottom sides.

8. The implant according to one of the preceding claims, **characterized in that** the plate (4) has a maximum thickness of approximately 6 mm to 8 mm.

9. The implant according to one of the preceding claims, **characterized in that** the plate (4) has a maximum width of 12 mm.

## Revendications

1. Implant pour la stabilisation d'une fracture du haut du bras dans la zone de tête du bras impliquant ou non le tubercule
- comportant une plaque (4),
- dans lequel sont pratiqués des alésages (12, 13, 14) pour des vis (5, 6, 7), la plaque présentant un guide (21, 22) pour une vis de tête de bras, qui maintient la vis de tête de bras de manière stable en angle par rapport à la plaque, **caractérisé en ce que** la plaque (4) présente un dispositif de maintien (27, 28, 31, 38) qui fixe de préférence de manière détachable la vis de tête de bras (8, 9) de manière stable pour empêcher un décalage dans le sens de la tige.

2. Implant selon la revendication 1, **caractérisé en ce que** la plaque (4) présente des guides (21, 22) pour de préférence deux vis de tête de bras (8, 9).

3. Implant selon la revendication 1 ou 2, **caractérisé en ce que** les guides sont des pièces alésées dont les sections transversales correspondent à un diamètre de tige (47) des vis de tête de bras (8, 9) .

4. Implant selon une des revendications précédentes, **caractérisé en ce que** l'angle (2, 3) est de 35° à 40° et de préférence de 37,5°.

5. Implant selon une des revendications précédentes, **caractérisé en ce que** la plaque présente comme dispositif de maintien entre les alésages (21, 22) des vis de tête de bras (8, 9) une fente (29) à alésage transversal (26).

6. Implant selon une des revendications précédentes, **caractérisé en ce que** les axes d'alésage (21, 22) des vis de tête de bras (8, 9) se situent dans un plan avec l'axe longitudinal de la tige de haut de bras.

7. Implant selon une des revendications précédentes, **caractérisé en ce que** la plaque (4) présente des alésages (42, 43) s'étendant parallèlement à son côté supérieur et inférieur.

8. Implant selon une des revendications précédentes, **caractérisé en ce que** la plaque (4) présente une épaisseur maximale d'environ 6 à 8 mm.

9. Implant selon une des revendications précédentes, **caractérisé en ce que** la plaque (4) présente une largeur maximale de 12 mm.
